# EUROPÄISCHE PATENTSCHRIFT

(11) **EP 2 674 186 B1**
(45) Veröffentlichungstag und Bekanntmachung des Hinweises auf die Patenterteilung: **29.12.2021**
(21) Anmeldenummer: 12004399.7
(22) Anmeldetag: 11.06.2012
(51) Int. Cl.: A61M 27/00

(54) **Fisteladaptervorrichtung**
Fistula adapter device
Dispositif d'adaptation de fistule

(43) Veröffentlichungstag der Anmeldung: 18.12.2013
(73) Patentinhaber: Carl Freudenberg KG, 69465 Weinheim (DE); Ruprecht-Karls-Universität Heidelberg, 69117 Heidelberg (DE)
(72) Erfinder: Büchler, Markus, 69120 Heidelberg (DE); Schneewind, Jörg, Farmington Hills, Michigan 48331 (US); Ryzko, Peter, 69514 Laudenbach (DE); Koeppen, Hannah, 64625 Bensheim-Auerbach (DE); Bork, Ulrich, 69120 Heidelberg (DE); Kenngott, Hannes Götz, 69120 Heidelberg (DE); Hoffmann, Wolf-Martin, 68163 Mannheim (DE); Kaul, Stefan, 69502 Hemsbach (DE); Weiß, Rainer, 69469 Weinheim (DE); Gramlich, Martin, 69493 Hirschberg (DE); Grafahrend, Dirk, 68165 Mannheim (DE)

(56) Entgegenhaltungen:
- WO-A1-01/85248
- WO-A1-97/45148
- WO-A1-2010/124844
- DE-U1-202004 018 245
- GB-A- 2 356 148
- US-A- 2 025 492
- US-A1- 2005 148 913
- US-A1- 2008 287 892
- US-A1- 2012 101 458

## Beschreibung

Die vorliegende Erfindung betrifft eine Fisteladaptervorrichtung zur Versorgung von enteroatmosphärischen Fisteln beim offenen Abdomen.

### Stand der Technik

Aus der Gebrauchsmusterschrift DE 20 2011 101 620 U1 ist eine Fisteldrainage zur Versorgung von enteroatmosphärischen Fisteln unter Verwendung der Vakuumtherapie zur Wundbehandlung bekannt. Aus dem Dokument US 2008/0287892 A1 ist eine Vorrichtung zur Versorgung von Fisteln ebenfalls bekannt.

### Darstellung der Erfindung

Die vorliegende Erfindung hat sich die Aufgabe gestellt, eine Fisteladaptervorrichtung mit einem modularen Aufbau bereitzustellen, die universell an individuelle Fistelformen anpassbar ist, die kein Vakuum benötigt und die damit die Gefahr von zusätzlichen Wundverletzungen sowie die Gefahr von sekundären Fistelbildungen vermeidet, so dass für den Patienten keine zusätzlichen Schmerzen auftreten.

Zudem soll die Fisteladaptervorrichtung auch in der Anwendung für das die Fisteln versorgende medizinische Personal besonders flexibel und möglichst einfach handhabbar sein.

Diese Aufgaben werden durch eine Fisteladaptervorrichtung gemäß Anspruch 1 gelöst.

Dazu umfasst die Fisteladaptervorrichtung zur Versorgung von enteroatmosphärischen Fisteln ohne Vakuum einen Schlauch zur Abführung der Fistelflüssigkeit mit variabler Schlauchhöhe und/oder variabler Schlauchquerschnittsfläche, eine am einen Schlauchende angeordnete Krempe als Wundauflagefläche mit variabler Querschnitts- bzw. Auflagefläche und/oder mit einer Gelschicht als Fistelauflage- oder angrenzende Fistelkontaktfläche und ein am anderen Schlauchende angeordnetes Abschlussstück zur Abdichtung und/oder Kopplung an andere medizinische Instrumente bzw. Vorrichtungen, wie beispielsweise einen Stromabeutel oder einen Flüssigkeitssammelbehälter.

Unter der Gelschicht wird eine dreidimensionale viskose, elastische oder halbfeste Masse verstanden, die formbar ist und sich beim Aufstülpen auf die Fistel bzw. bei Kontakt mit der Fistel mit geringer mechanischer Belastung dicht auf diese auflegt oder an diese angrenzt und sich somit individuell an die Fistelgeometrie anpasst, ohne die Wunde zusätzlich zu verletzen bzw. ohne, dass neue Fisteln gebildet werden. Die Gelschicht kann dabei aus organischem oder anorganischem Material sein.

Des Weiteren weist die Gelschicht inhärent eine adhäsive Wirkung auf bzw. ist die Gelschicht adhäsiv, so dass das ansonsten übliche Anlegen eines zusätzlichen Vakuums, beispielsweise im Rahmen einer Vakuumtherapie, insbesondere für ein genaues Platzieren und dichtes Auflegen gerade nicht nötig ist. Dies ist für den Patienten von Vorteil, da das Anlegen eines Vakuums zumindest häufig als unangenehm oder sogar als schmerzhaft empfunden wird.

Ferner ist die Gelschicht bevorzugt aus einem medizinisch zugelassenen organischen oder anorganischen Gel, insbesondere aus einem biokompatiblen Gel, gebildet.

Alternativ oder kumulativ zu der Gelschicht weist die Krempe als Wundauflagefläche bzw. als Adapter zur Wunde eine einstellbare variable Querschnittsfläche auf, um sich individuell, flexibel und großflächig auf die Wunde auflegen zu können.

Die an dem Schlauch angeordnete Krempe umgibt das eine Ende des Schlauchs als seitlich abstehendes flächiges Gebilde, vergleichbar mit der Krempe eines Huts, und hat eine flexible symmetrische oder asymmetrische Form.

Durch die einstellbare variable Höhe und/oder einstellbare variable Querschnittsfläche des Schlauchs zur Abführung der Fistelflüssigkeit und/oder die einstellbare variable Querschnitts- bzw. Auflagefläche der Krempe ist die Fisteladaptervorrichtung für das medizinische Personal besonders flexibel einsetzbar.

In einer erfindungsgemäßen Ausgestaltung ist der Schlauch zur Abführung der Fistelflüssigkeit ein Teleskoprohr. Das Teleskoprohr besteht aus mehreren zylindrisch verjüngenden und parallel in sich geführten Röhren oder Hohlzylindern, welche linear bis zum Anschlag auf eine maximale Auszugslänge herausgezogen und wieder ineinander zurückgeschoben werden können.

In einer alternativen erfindungsgemäßen Ausgestaltung ist der Schlauch zur Abführung der Fistelflüssigkeit ein Faltenbalg. Als Faltenbalg wird hier ein elastischer, sich "zieharmonikaartig" zusammenfaltender Schlauch verstanden. Um den Rückfluss der Fistelflüssigkeit zu unterbinden, weist die Fisteladaptervorrichtung vorzugsweise im Schlauch und/oder im Abschlussstück angeordnet zumindest ein Rückflussventil, gegebenenfalls mit Pumpwirkung, auf.

Erfindungsgemäß sind der Schlauch und die Krempe aus einem flexiblen Werkstoff gefertigt, bevorzugt aus einem Standard-Elastomer oder aus einem thermoplastischen Elastomer.

Als Standard-Elastomere kommen hier beispielsweise Naturkautschuk oder Silikonkautschuk oder Silikonelastomere, wie Methyl-Vinyl-Silikonkautschuk (VQM), Fluorsilikon-Kautschuk (FMQ), in Betracht, aber auch Styrolbutadienkautschuk (SBR), Isorprenkautschuk (IR), Ethylen-Propylen-Dien-Kautschuk (EPDM), Polynorbonen (PNR), Acrylnitril-Butadienkautschuk (NBR), Butylkautschuk (IIR), Chlorbutadien-Kautschuk, Acrylat-Kautschuk (ACM), Fluorkautschuk (FPM), Ethylen-Acrylat-Kautschuk (AEM) oder Butadien-Kautschuk (BR).

Unter die thermoplastischen Elastomere fallen sowohl Blockcopolymere als auch Elastomerlegierungen bzw. Polyblends. Dazu gehören beispielsweise unvernetzte oder vernetzte thermoplastische Elastomere auf Olefinbasis (TPE-O, TPE-V), thermoplastische Elastomere auf Urethanbasis (TPE-U), thermoplastische Polyesterelastomere oder thermoplastische Copolyester (TPE-E), Styrol-Blockcopolymere (TPE-S), thermoplastische Copolyamide (TPE-A), Blends auf Acrylatbasis, Blends auf Fluorkautschukbasis und andere thermoplastische Vulkanisate.

Dabei sind vorteilhafterweise zumindest die wundseitigen Oberflächen der Krempe und/oder die (fistelflüssigkeitsseitigen) Innenflächen des Schlauchs aus einem medizinisch zugelassenen Material, insbesondere biokompatibel.

Bedingt durch das Material und/oder die Form weist die Krempe eine besonders gute adhäsive Wirkung auf, d.h. ist besonders adhäsiv, was insbesondere für ein genaues Platzieren ohne Verrutschen von Vorteil ist bei gleichzeitiger geringer mechanischer Belastung der Wunde.

Vorteilhafterweise ist das Abschlussstück, insbesondere als weiterer Adapter der Fisteladaptervorrichtung, aus einem Thermoplasten gefertigt. Zu den Thermoplasten zählen z.B. Acrylnitril-Butadien-Styrol (ABS), Polyamide (PA), Polylactat (PLA), Polymethylmethacrylat (PMMA), Polycarbonat (PC), Polyethylenterephthalat (PET), Polyethylen (PE), Polypropylen (PP), Polystyrol (PS), Polyetheretherketon (PEEK).

Dabei sind vorteilhafterweise zumindest die (fistelflüssigkeitsseitigen) Innenflächen des Abschlussstücks, insbesondere für andere medizinische Instrumente bzw. Vorrichtungen, wie beispielsweise einen Stromabeutel oder einen Flüssigkeitssammelbehälter, aus einem medizinisch zugelassenen Material, insbesondere biokompatibel.

Die Zertifizierung der Biokompatibilität der medizinischen Werkstoffe und Produkte erfolgt dabei jeweils nach ISO 10993 1-20.

Der Schlauch und die Krempe können aus dem gleichen Material, insbesondere aus dem gleichen (thermoplastischen) Elastomer, gefertigt sein, um den Herstellprozess und die spätere Entsorgung der Fisteladaptervorrichtung zu vereinfachen.

Erfindungsgemäß ist die Krempe flexibler als der Schlauch ausgestaltet, um insbesondere die mechanische Belastung der Wunde zu verringern. Die besonders flexible Ausgestaltung wird vorzugsweise entweder über das entsprechend flexiblere Material oder über die entsprechende Geometrie, zum Beispiel eine geringere Dicke, erreicht. Der Schlauch ist in diesem Fall bevorzugt aus einem vergleichsweise steife(re)n Material gefertigt, um eine besonders stabile Abführung der Fistelflüssigkeit zu gewährleisten.

Für eine besonders gute bzw. optimale Wundkontrolle sind zumindest die Krempe und die optionale Gelschicht der Fisteladaptervorrichtung transparent ausgestaltet, insbesondere durch eine entsprechende Auswahl der Materialien.

In bevorzugter Ausgestaltung der Fisteladaptervorrichtung sind die Krempe und die optionale Gelschicht mit Wundadditiven versetzt. Als Wundadditive werden hier Zusätze von Medikamenten und/oder wundheilungsfördernden Mitteln verstanden.

Um die Wechselintervalle der Fisteladaptervorrichtung zu verlängern, sind die Krempe, die optionale Gelschicht, der Schlauch und/oder das Abschlussstück bevorzugt mit antimikrobiellen Additiven versetzt.

Unter einem antimikrobiellen Additiv wird hier ein Stoff verstanden, der die Vermehrungsfähigkeit oder Infektiosität von Mikroorganismen reduziert oder sie abtötet beziehungsweise inaktiviert. Zu den antimikrobiellen Additiven gehören Antibiotika gegen Bakterien und die Antimykotika gegen Pilze und pathogene Hefen. Des Weiteren werden alle Antiparasitika zu den antimikrobiellen Substanzen gerechnet, zu denen wiederum die Antihelminthika gegen parasitäre Würmer und die Antiprotozoika gegen pathogene Amöben gerechnet werden. Neben diesen Substanzgruppen, die der unmittelbaren spezifischen Therapie dienen, zählen auch alle Desinfektionsmittel zu den antimikrobiellen Additiven. Diese können neben den oben genannten Erregern auch Viren inaktivieren.

Für die Lagerung bzw. den Transport der Fisteladaptervorrichtung ist vorzugsweise zumindest die Gelschicht der Fisteladaptervorrichtung mit einer abziehbaren Folie überzogen und damit insbesondere an der Krempe fixiert. Gleichzeitig kann eine entsprechende Folie insgesamt vor Umwelteinflüssen, wie zum Beispiel vor Schmutz, Feuchtigkeit, Licht und/oder Wärme, schützen. Unmittelbar vor Gebrauch der Fisteladaptervorrichtung zur Versorgung der Fisteln kann diese Folie von dem medizinischen Personal durch einfaches Abziehen leicht entfernt werden.

Zur Erhöhung der Stabilität unter gleichzeitigem Erhalt der Flexibilität der vorzugsweise eingesetzten Gelschicht, insbesondere nach dem Abziehen der genannten Fixierungs- bzw. Schutzfolie, umfasst die Gelschicht als Gel bevorzugt ein Polymer, das durch Einwirkung von Feuchtigkeit, Licht, insbesondere UV-Licht, und/oder Wärme (weiter) vernetzt bzw. fester wird.

Um die Handhabbarkeit der Fisteladaptervorrichtung für das medizinische Personal zu vereinfachen, ist der Schlauch an der Außensichtseite und/oder die Krempe an der Außensichtseite mit Höhen-, Querschnittsflächen- bzw. Auflageflächenmarkierungen, beispielsweise durch konzentrische Kreise mit Millimeter-Angabe, versehen, zum Beispiel als optische Schnitt- oder Einstellhilfe bezüglich der Höhe bzw. des Querschnitts.

### Kurzbeschreibung der Zeichnungen

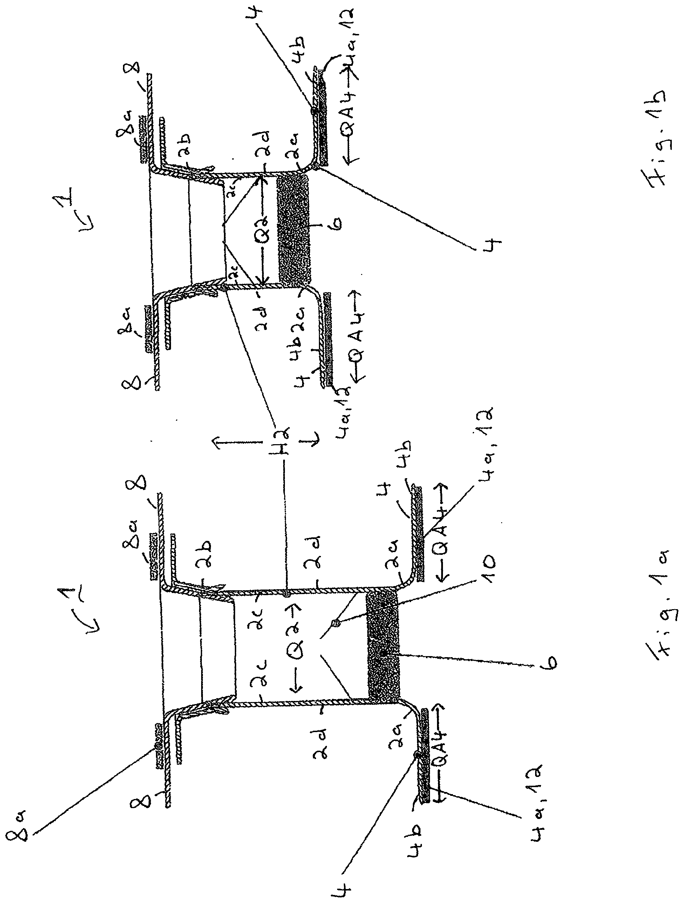

- Figuren 1a, 1b: je eine schematische Fisteladaptervorrichtung mit unterschiedlicher Höhe des Schlauchs (nicht maßstabgetreu)

### Ausführung der Erfindung

Der Gegenstand der Erfindung wird nachfolgend anhand eines Beispiels näher erläutert, ohne die Erfindung einzuschränken.

In Figur 1a und 1b ist jeweils eine Fisteladaptervorrichtung 1 gezeigt zur Versorgung von nicht dargestellten enteroatmosphärischen Fisteln ohne Vakuum umfassend, einen flexiblen Schlauch 2 zur Abführung der Fistelflüssigkeit mit variabler Höhe H2 und variabler Querschnittsfläche Q2, eine am einen Schlauchende 2a angeordnete Krempe 4 als Wundauflagefläche mit variabler Querschnitts- bzw. Auflagefläche QA4 und mit einer Gelschicht 6 als Fistelauflage- oder angrenzende Fistelkontaktfläche und ein am anderen Schlauchende 2b angeordnetes Abschlussstück 8 zur Abdichtung und/oder Kopplung an andere in den Figuren 1a,1b nicht dargestellte medizinische Vorrichtungen, wie beispielsweise einen Stromabeutel oder einen Flüssigkeitssammelbehälter.

Die Gelschicht 6 ist bevorzugt aus einem biokompatiblen Gel gebildet.

In den Figuren 1a und 1b weist die Krempe 4 als Wundauflagefläche bzw. als Adapter zur nicht dargestellten Wunde eine variable Querschnitts- bzw. Auflagefläche QA4 auf, um sich individuell, flexibel und großflächig auf die Wunde auflegen zu können.

Die an dem Schlauch 2 angeordnete Krempe 4 umgibt das Schlauchende 2a als seitlich abstehendes flächiges Gebilde, vergleichbar mit der Krempe eines Huts, und hat eine flexible symmetrische oder asymmetrische Form.

Durch die variable Höhe H2 und die variable Querschnittsfläche Q2 des Schlauchs 2 zur Abführung der Fistelflüssigkeit und die variable Querschnitts- bzw. Auflagefläche QA4 der Krempe 4 ist die Fisteladaptervorrichtung 1 für das medizinische Personal besonders flexibel und individuell einsetzbar.

Erfindungsgemäß ist der Schlauch 2 zur Abführung der Fistelflüssigkeit ein nicht in den Figuren 1a und 1b dargestelltes Teleskoprohr oder alternativ ein Faltenbalg. Die Figuren 1a und 1b zeigen die gleiche Ausführung mit lediglich unterschiedlich eingestellter variabler Höhe H2 des Schlauchs 2, nämlich Figur 1a eine bezüglich der Höhe länger eingestellte Variante und Figur 1b eine bezüglich der Höhe kürzer eingestellte Variante des Schlauchs 2.

Um den Rückfluss der Fistelflüssigkeit zu unterbinden, weist die Fisteladaptervorrichtung 1 vorzugsweise im Schlauch 2 angeordnet Rückflussventile 10, gegebenenfalls mit Pumpwirkung, auf, wie in Figur 1a gezeigt.

Vorteilhafterweise sind der Schlauch 2 und/oder die Krempe 4 aus einem Standard-Elastomer oder einem thermoplastischen Elastomer gefertigt. Dabei sind vorteilhafterweise zumindest die wundseitigen Oberflächen 4a der Krempe 4 und die fistelflüssigkeitsseitigen Innenflächen 2c des Schlauchs 2 biokompatibel.

Bedingt durch das Material und/oder die Form weist die Krempe 4 wie die Gelschicht eine besonders gute adhäsive Wirkung auf, d.h. ist besonders adhäsiv, was insbesondere für ein genaues Platzieren ohne Verrutschen von Vorteil ist bei gleichzeitiger geringer mechanischer Belastung der Wunde. Vorteilhafterweise ist das Abschlussstück 8, insbesondere als weiterer Adapter der Fisteladaptervorrichtung 1, aus einem Thermoplasten gefertigt.

Dabei sind vorteilhafterweise zumindest die fistelflüssigkeitsseitigen Innenflächen 8a des Abschlussstücks 8, insbesondere für andere nicht dargestellte medizinische Vorrichtungen, wie beispielsweise einen Stromabeutel oder einen Flüssigkeitssammelbehälter, biokompatibel.

Der Schlauch 2 und die Krempe 4 können aus dem gleichen Material, insbesondere aus dem gleichen (thermoplastischen) Elastomer, gefertigt sein, um den Herstellprozess und die spätere Entsorgung der Fisteladaptervorrichtung 1 zu vereinfachen.

Erfindungsgemäß ist die Krempe 4 flexibler als der Schlauch 2 ausgestaltet, um insbesondere die mechanische Belastung der Wunde zu verringern. Die besonders flexible Ausgestaltung wird vorzugsweise entweder über das entsprechend flexiblere Material oder über die entsprechende Geometrie, zum Beispiel eine geringere Dicke, erreicht. Der Schlauch 2 ist in diesem Fall bevorzugt aus einem vergleichsweise steife(re)n Material gefertigt, um eine besonders stabile Abführung der Fistelflüssigkeit zu gewährleisten.

Für eine besonders gute bzw. optimale Wundkontrolle sind die Krempe 4 und die Gelschicht 6 der Fisteladaptervorrichtung 1 transparent ausgestaltet, insbesondere durch eine entsprechende Auswahl der Materialien.

In bevorzugter Ausgestaltung der Fisteladaptervorrichtung 1 ist zumindest die Krempe 4 mit zumindest einem Wundadditiv 12 versetzt. Als Wundadditive werden hier Zusätze von Medikamenten und/oder wundheilungsfördernden Mitteln verstanden.

Um die Wechselintervalle der Fisteladaptervorrichtung 1 zu verlängern, sind die Krempe 4, die Gelschicht 6, der Schlauch 2 und/oder das Abschlussstück 8 bevorzugt mit nicht dargestellten antimikrobiellen Additiven versetzt.

Für die Lagerung bzw. den Transport der Fisteladaptervorrichtung 1 ist zumindest die Gelschicht 6 der Fisteladaptervorrichtung 1 vorzugsweise mit einer nicht dargestellten abziehbaren Folie überzogen und damit insbesondere an der Krempe 4 fixiert. Gleichzeitig kann eine entsprechende Folie insgesamt vor Umwelteinflüssen, wie zum Beispiel vor Schmutz, Feuchtigkeit, Licht und/oder Wärme, schützen. Unmittelbar vor Gebrauch der Fisteladaptervorrichtung 1 zur Versorgung der Fisteln kann diese Folie von dem medizinischen Personal durch einfaches Abziehen leicht entfernt werden.

Zur Erhöhung der Stabilität unter gleichzeitigem Erhalt der Flexibilität der Gelschicht 6, insbesondere nach dem Abziehen der genannten nicht in den Figuren 1a und1b gezeigten Fixierungs- bzw. Schutzfolie, umfasst die Gelschicht 6 als Gel bevorzugt ein Polymer, das durch Einwirkung von Feuchtigkeit, Licht, insbesondere UV-Licht, und/oder Wärme (weiter) vernetzt bzw. fester wird.

Um die Handhabbarkeit der Fisteladaptervorrichtung 1 für das medizinische Personal zu vereinfachen, ist der Schlauch 2 an der Außensichtseite 2d und/oder die Krempe 4 an der Außensichtseite 4b mit nicht in den Figuren 1a und 1b dargestellten Höhen-, Querschnittsflächen- bzw. Auflageflächenmarkierungen, beispielsweise durch konzentrische Kreise mit Millimeter-Angabe, versehen, zum Beispiel als optische Schnitt- oder Einstellhilfe bezüglich der Höhe bzw. des Querschnitts.

### Bezugszeichenliste

- 1: Fisteladaptervorrichtung
- 2: Schlauch
- 2a: Schlauchende an der Krempe (4)
- 2b: Schlauchende am Abschlussstück (8)
- 2c: Innenflächen des Schlauchs (2)
- 2d: Außensichtseite des Schlauchs (2)
- 4: Krempe
- 4a: wundseitige Oberflächen der Krempe (4)
- 4b: Außensichtseite der Krempe (4)
- 6: Gelschicht
- 8: Abschlussstück
- 8a: Innenflächen des Abschlussstücks (8)
- 10: Rückflussventil
- 12: Wundadditiv

- H2: variable Höhe des Schlauchs (2)
- Q2: variabler Querschnitt des Schlauchs (2)
- QA4: variable Querschnitts- bzw. Auflagefläche der Krempe (4)

## Patentansprüche

1. Fisteladaptervorrichtung (1) zur Versorgung von enteroatmosphärischen Fisteln ohne Vakuum umfassend, einen Schlauch (2) zur Abführung der Fistelflüssigkeit mit variabler Höhe (H2) und/oder variabler Querschnittsfläche (Q2), eine am einen Schlauchende (2a) angeordnete Krempe (4) als Wundauflagefläche mit variabler Querschnitts- bzw. Auflagefläche (QA4) und/oder mit einer Gelschicht (6) als Fistelauflage- oder angrenzende Fistelkontaktfläche und ein am anderen Schlauchende (2b) angeordnetes Abschlussstück (8) zur Abdichtung und/oder Kopplung an andere medizinische Vorrichtungen, wobei der Schlauch (2) und die Krempe (4) aus einem flexiblen Werkstoff, bevorzugt aus Standard-Elastomeren oder aus thermoplastischen Elastomeren gefertigt sind, wobei die Krempe (4) flexibler ist als der Schlauch (2) ist, **dadurch gekennzeichnet, dass** der Schlauch (2) als Teleskoprohr oder als Faltenbalg ausgestaltet ist.

2. Fisteladaptervorrichtung (1) nach Anspruch 1, bei dem im Schlauch (2) und/oder im Abschlussstück (8) zumindest ein Rückflussventil (10) angeordnet ist.

3. Fisteladaptervorrichtung (1) nach einem der vorhergehenden Ansprüche, bei dem die Gelschicht (6) aus einem medizinisch zugelassenen Gel gebildet ist, insbesondere biokompatibel ist.

4. Fisteladaptervorrichtung (1) nach einem der vorhergehenden Ansprüche, bei dem zumindest die wundseitigen Oberflächen (4a) der Krempe (4) und/oder die Innenflächen (2c) des Schlauchs (2) aus einem medizinisch zugelassenen Material, insbesondere biokompatibel, sind.

5. Fisteladaptervorrichtung (1) nach einem der vorhergehenden Ansprüche, bei dem das Abschlussstück (8) aus Thermoplasten gefertigt ist, wobei zumindest die Innenflächen (8a) des Abschlussstücks (8) aus einem medizinisch zugelassenen Material, insbesondere biokompatibel, sind.

6. Fisteladaptervorrichtung (1) nach einem der vorhergehenden Ansprüche, bei dem der Schlauch (2) und die Krempe (4) aus dem gleichen Material, insbesondere aus dem gleichen (thermoplastischen) Elastomer, gefertigt sind.

7. Fisteladaptervorrichtung (1) nach einem der vorhergehenden Ansprüche, bei dem die Krempe (4) und die optionale Gelschicht (6) transparent sind.

8. Fisteladaptervorrichtung (1) nach einem der vorhergehenden Ansprüche, bei dem die Krempe (4) und die optionale Gelschicht (6) mit Wundadditiven (12) versetzt sind.

9. Fisteladaptervorrichtung (1) nach einem der vorhergehenden Ansprüche, bei dem die Krempe (4), die Gelschicht (6), der Schlauch (2) und/oder das Abschlussstück (8) mit antimikrobiellen Additiven versetzt sind.

10. Fisteladaptervorrichtung (1) nach einem der vorhergehenden Ansprüche, bei dem die Gelschicht (6) für die Lagerung bzw. den Transport mit einer abziehbaren Folie überzogen ist.

11. Fisteladaptervorrichtung (1) nach einem der vorhergehenden Ansprüche, bei dem die Gelschicht (6) als Gel ein Polymer umfasst, das durch Einwirkung von Feuchtigkeit, Licht, insbesondere UV-Licht, und/oder Wärme (weiter) vernetzt bzw. fester wird.

12. Fisteladaptervorrichtung (1) nach einem der vorhergehenden Ansprüche, bei dem der Schlauch (2) an der Außensichtseite (2d) und/oder die Krempe (4) an der Außensichtseite (4b) mit Höhen-, Querschnittsflächen- bzw. Auflageflächenmarkierungen versehen sind.

## Claims

1. Fistula adapter device (1) for the management of enteroatmospheric fistulas without vacuum assistance, comprising a tube (2) which serves to remove the fistula liquid and is of variable height (H2) and/or variable cross-sectional area (Q2), a rim (4) which is arranged at one end (2a) of the tube and serves as a wound contact surface of variable cross-sectional area or contact area (QA4), and/or a gel layer (6) as fistula support surface or adjoining fistula contact surface, and an endpiece (8) which is arranged at the other end (2b) of the tube and serves for sealing and/or coupling to other medical devices, wherein the tube (2) and the rim (4) are produced from a flexible material, preferably from standard elastomers or from thermoplastic elastomers, wherein the rim (4) is more flexible than the tube (2), **characterized in that** the tube (2) is designed as a telescopic tube or as a bellows.

2. Fistula adapter device (1) according to Claim 1, in which at least one nonreturn valve (10) is arranged in the tube (2) and/or in the endpiece (8).

3. Fistula adapter device (1) according to one of the preceding claims, in which the gel layer (6) is formed from a medically approved gel and in particular is biocompatible.

4. Fistula adapter device (1) according to one of the preceding claims, in which at least the wound-side surfaces (4a) of the rim (4) and/or the inner surfaces (2c) of the tube (2) are of a medically approved material and in particular are biocompatible.

5. Fistula adapter device (1) according to one of the preceding claims, in which the endpiece (8) is produced from thermoplastics, wherein at least the inner surfaces (8a) of the endpiece (8) are of a medically approved material and in particular are biocompatible.

6. Fistula adapter device (1) according to one of the preceding claims, in which the tube (2) and the rim (4) are produced from the same material, in particular from the same (thermoplastic) elastomer.

7. Fistula adapter device (1) according to one of the preceding claims, in which the rim (4) and the optional gel layer (6) are transparent.

8. Fistula adapter device (1) according to one of the preceding claims, in which wound additives (12) are added to the rim (4) and to the optional gel layer (6) .

9. Fistula adapter device (1) according to one of the preceding claims, in which antimicrobial additives are added to the rim (4), the gel layer (6), the tube (2) and/or the endpiece (8).

10. Fistula adapter device (1) according to one of the preceding claims, in which the gel layer (6) is covered with a peel-off film for storage or transport.

11. Fistula adapter device (1) according to one of the preceding claims, in which the gel layer (6) comprises as gel a polymer, which is (further) crosslinked or strengthened by the action of moisture, light, in particular UV light, and/or heat.

12. Fistula adapter device (1) according to one of the preceding claims, in which the outer visible side (2d) of the tube (2) and/or the outer visible side (4b) of the rim (4) are provided with height makings, cross-sectional area markings or contact surface markings.

## Revendications

1. Dispositif adaptateur de fistule (1) pour le traitement de fistules entéro-atmosphériques sans vide, comprenant un tuyau (2) pour l'évacuation du liquide de fistule ayant une hauteur (H2) variable et/ou une surface de section transversale (Q2) variable, un bord (4) agencé à une extrémité de tuyau (2a) en tant que surface d'appui sur la plaie ayant une surface de section transversale ou d'appui (QA4) variable et/ou ayant une couche de gel (6) en tant que surface d'appui sur la fistule ou surface de contact avec la fistule adjacente, et un tronçon de fermeture (8) agencé à l'autre extrémité de tuyau (2b) pour l'étanchéification et/ou l'accouplement à d'autres dispositifs médicaux, le tuyau (2) et le bord (4) étant fabriqués en un matériau flexible, de préférence en élastomères standard ou en élastomères thermoplastiques, le bord (4) étant plus flexible que le tuyau (2), **caractérisé en ce que** le tuyau (2) est conçu sous la forme d'un tube télescopique ou sous la forme d'un soufflet.

2. Dispositif adaptateur de fistule (1) selon la revendication 1, dans lequel au moins une soupape de reflux (10) est agencée dans le tuyau (2) et/ou dans le tronçon de fermeture (8).

3. Dispositif adaptateur de fistule (1) selon l'une quelconque des revendications précédentes, dans lequel la couche de gel (6) est formée en un gel médicalement autorisé, notamment est biocompatible.

4. Dispositif adaptateur de fistule (1) selon l'une quelconque des revendications précédentes, dans lequel au moins les surfaces côté plaie (4a) du bord (4) et/ou les surfaces intérieures (2c) du tuyau (2) sont en un matériau médicalement autorisé, notamment sont biocompatibles.

5. Dispositif adaptateur de fistule (1) selon l'une quelconque des revendications précédentes, dans lequel le tronçon de fermeture (8) est fabriqué en thermoplastiques, au moins les surfaces intérieures (8a) du tronçon de fermeture (8) étant en un matériau médicalement autorisé, notamment étant biocompatibles.

6. Dispositif adaptateur de fistule (1) selon l'une quelconque des revendications précédentes, dans lequel le tuyau (2) et le bord (4) sont fabriqués en le même matériau, notamment en le même élastomère (thermoplastique).

7. Dispositif adaptateur de fistule (1) selon l'une quelconque des revendications précédentes, dans lequel le bord (4) et la couche de gel optionnelle (6) sont transparents.

8. Dispositif adaptateur de fistule (1) selon l'une quelconque des revendications précédentes, dans lequel le bord (4) et la couche de gel optionnelle (6) sont mélangés avec des additifs pour plaie (12).

9. Dispositif adaptateur de fistule (1) selon l'une quelconque des revendications précédentes, dans lequel le bord (4), la couche de gel (6), le tuyau (2) et/ou le tronçon de fermeture (8) sont mélangés avec des additifs antimicrobiens.

10. Dispositif adaptateur de fistule (1) selon l'une quelconque des revendications précédentes, dans lequel la couche de gel (6) est recouverte d'un film amovible pour le stockage ou le transport.

11. Dispositif adaptateur de fistule (1) selon l'une quelconque des revendications précédentes, dans lequel la couche de gel (6) comprend en tant que gel un polymère, qui est (en outre) réticulé ou solidifié par exposition à l'humidité, à la lumière, notamment à la lumière UV, et/ou à la chaleur.

12. Dispositif adaptateur de fistule (1) selon l'une quelconque des revendications précédentes, dans lequel le tuyau (2) sur le côté visible extérieur (2d) et/ou le bord (4) sur le côté visible extérieur (4b) sont munis de marquages de hauteur, de surface de section transversale ou de surface d'appui.
